# EUROPEAN PATENT APPLICATION

(11) **EP 0 814 090 A1**
(43) Date of publication of application: **29.12.1997**
(21) Application number: 97103408.7
(22) Date of filing: 12.06.1990
(51) Int. Cl.: C07K 14/15, G01N 33/569, G01N 33/68, A61K 39/21

(54) **Peptides and antibodies derived therefrom for the diagnosis of, therapy for and vaccination against htlv-1 infection**

(30) Priority: 13.06.1989 SE 8902148
(71) Applicant: SYNTELLO INC, San Diego, CA 92121 (US)
(72) Inventor: Svennerholm, Bo, 412 68 Göteborg (SE); Rymo, Lars, 430 80 Hovas (SE); Horal, Peter, 412 66 Göteborg (SE); Jeansson, Stig, 411 27 Göteborg (SE); Vahlne, Anders, 430 80 Hovas (SE)
(74) Representative: Weller, Wolfgang, Dr.rer.nat.

(57) **Abstract**

Peptides corresponding to regions of proteins encoded by the HTLV-1 genome are provided. These peptides are immunologically reactive with HTLV-1 specific antibodies. Several of the peptides are capable of distinguishing between HTLV-1 and HTLV-2 infection. The peptides are useful in assays for detection of HTLV-1 infection or exposure and in compositions to elicit the production of antibodies against HTLV-1 in animals and man. Antibodies elicited in response to the antibodies are also provided.

## Description

### Background of the invention

The present invention relates to peptides, the sequences of which correspond to antigenic regions of an immunologically important protein of HTLV-1. These peptides are useful as diagnostic reagents for detecting the presence of antibodies to HTLV-1 and may also be useful as immunogens in compositions and methods to elicit antibodies against HTLV-1 in animals and man.

The etiologic agent of adult T-cell leukemia/lymphoma (ATL) has been identified as HTLV-1 (human T-cell lymphotropic virus type 1.). See e.g., Sarngadharan et.al., "Human T-cell Leukemia Viruses", in: Virology, B. N. Fields et. al., eds., pp. 1345-1371 (1985), for a review. The region of the world where the virus is most prevalent is the island of Kyushu in southern Japan where about 15 % of the population has been infected. Recently a tropical paralysis called tropical spastic paraparesis (TSP) has also been associated with HTLV-1 infection. Rodgers-Johnson et.al., "HTLV-1 and HTLV-III and Tropical Spastic Paraperesis ", Lancet, II-1247 (1985); Vernant et.al., "Endemic Tropical Spastic Paraperesis Associated With Human T-Lymphotropic Virus Type I: A Clinical Seroepidemiological Study of 25 Cases", Ann. Neurol., 21:123 (1987). In the tropics TSP is of the same magnitude and importance as the multiple sclerosis syndrome is in the western world. Marx, "Leukemia Virus Linked to Nerve Disease", Science, 236;1059-1061 (1987).

HTLV-1 and Human T cell Lymphotropic Virus Type II (HTLV-2) are antigenically related members of a family of oncogenic retroviruses sharing a tropism for T lymghocytes and a correlation with lymphoproliferative diseases. Due to the degree of homology between HTLV-2 and HTLV-1, serological studies have been unable to differentiate between infection by HTLV-1 and HTLV-2. Unequivocal differentiation of HTLV-1 and HTLV-2 requires virus isolation and/or molecular identification. Although HTLV-2 has not been definitely associated with human disease, intravenous drug abusers (IVDAs) previously thought to be infected with HTLV-1 have now been found to be infected with HTLV-2. It is now thought that HTLV-2 infection may be quite common among IVDAs. Tedder et.al., "Low Prevalance in UK of HTLV-1 and HTLV-II Infection in Subjects With AIDS, With Extended Lymphodenopathy, and at Risk of Aids", Lancet, 2:125-128; Robert-Gurott et.al., "Prevalence of Antibodies to HTLV-1, -II and -III in Intravenous Drug Abusers from an AIDS Endemic Region", J.A.M.A., 255:3133-3137 (1986). In view of the association of HTLV-2 with AIDS, virus isolation is particulary difficult and dangerous. Hence, it would be useful to be able to distinguish between HTLV-1 and HTLV-2 infections without resorting to virus isolation and manipulation. An automated blood screening test format capable of readily detecting HTLV-1 and distinguishing HTLV-1 from HTLV-2 infections is also critical to a supply of uninfected blood.

Methods for detection of HTLV-1 infection, in general, measure exposure to the virus by detecting and quantifying antibodies to HTLV-1 antigens in blood, sera, and blood-derived products. Such assays can be used to aid diagnosis of ATL and TSP and to screen blood and blood products for previous exposure to HTLV-1 thereby preventing further infections.

The current attempts to diagnose HTLV-1 infections and screen for exposure to HTLV-1 include enzyme-linked immunosorbent assay (ELISA) techniques to detect the presence of antibodies to immunogenic components of HTLV-1 in a test sample. Other methods may utilize Western blatting techniques to detect HTLV-1 specific antibodies in test samples.

In general, almost any known immunoassay, including but not limited to radioimmunoassays, agglutination tests or indirect immunoflourescence, in addition to ELISA and Western blots, can be adapted, by use of specific reagents, for the detection of HTLV-1 a and antibodies thereto.

The source of antigens for these assays may include but are not limited to antigenic proteins obtained from HTLV-1 infected T cell lines and antigens produced by recombinant DNA techniques. The use of antigens obtained from these sources, however, has significant drawbacks.

The production of HTLV-1 per se in continuous cell lines must be performed in high risk (P3 containment) laboratories due the danger to investigators who may become adversely exposed to the virus. It is also likely that the use of T cell derived HTLV-1 antigens can produce false negative and false positive results in ELISA tests. For example, by analogy, in measuring esposure to the AIDS virus, there have been false negative and false positive results reported with ELISA tests using whole virus HIV-1 antigens obtained from cell lines. Gurtler et.al., "Sensitivity and Specificity of Commercial ELISA kits For Screening Anti-LAV/HTLV-III". J. Virol. Met., 15:11-23 (1987). Western blot analyses for HTLV-1 detection, using electroblotted whole virus antigens, should provide greater specificity but are more laborious and time-consuming than ELISA tests. Further-more, since HTLV-1 producing cells are of human origin, viral antigen preparations obtained from these cells lines, unless exhaustively purified, may be contaminated with normal cellular antigens, such as HLA antigens, which could produce false positive reactions in a ELISA test.

Exhaustive purification of viral antigens from cell lines can also conceivably destroy immunogenicity of immunologically important proteins or otherwise inactivate antigens, thereby producing reagents that result in false negative reactions. In addition, false negative reactions using live-virus-derived antigens may occur because of steric hindrance whereby antibodies cannot react with their specific antigens because the reaction is blocked by the presence of other antigens and antibodies in the reaction mixture. Also, proteins isolated from live virus can be unsuitable for vaccination due to the risk of contamination by whole virus or viral genetic material.

ELISA tests to detect HTLV-1 infection may also employ immunologically important viral proteins produced by cloning portions of the HTLV-1 genome in bacteria. The complete nucleotide sequence of HTLV-1 has been reported by Seiki et.al., "Human Adult T-cell Leukemia Virus: Complete Nucleotide Sequence of the Provirus Genome Integrated in Leukemia Cell DNA", Proc. Nat. Acad. Sci. USA, 80:3618-3622 (1983). The viral envelope glycoproteins and core proteins, respectively encoded by the env and gag genes of HTLV-1, are apparently the antigens recognized by antibodies in the sera of patients with HTLV-1 infection.

Immunologically important HTLV-1 antigens, which are present in the viral envelope and core, may be prepared by cloning portions of the HTLV-1 genome in various expression systems such as bacteria, yeast or vaccinia. Such recombinant antigens may be used in diagnosis and as potential vaccine compositions as has been done for HIV-1 proteins. See, e.g. Cabradilla et.al., "Serodiagnosis of Antibodies to the Human AIDS Retrovirus With a Bacterially Synthesized env Polypeptide", Biotechnology, 4:128-133 (1986); Chang et. al., "Detection of Antibodies to Human T-cell Lymphotropic Virus-III (HTLV-III) With an Immunoassay Employing a Recombinant Escherichia coli - Derived Viral Antigenic Peptide", Bio-/technology, 3:905-909 (1985); Putney et.al., "HTLV-III/LAV-Neutralising Antibodies to an E. Coli -Produced Fragment of the Virus Envelope", Science, 234:1392-1395 (1986); Kieny et.al. "AIDS Virus env Protein Expressed from a Recombinant Vaccinia Virus", Bio/technology, 4:790-795 (1986). HTLV-1 antigens produced by recombinant DNA methods, however, must still be exhaustively purified to avoid false positive reactions in the ELISA due to any antibody reactivity to antigens of the expression system which may contaminate the HTLV-1 antigen preparation. Also, denaturation of HTLV-1 antigens during purification may destroy important antigen activity.

In the case of vaccines, recombinant proteins purified from bacteria or yeast are often contaminated with bacterial or yeast proteins. Even minute amounts of these contaminants are capable of causing adverse reactions.

While HTLV-1 antigens produced by recombinant techniques may be an improvement over antigens obtained from virus-infected cell cultures, the recombinant proteins still may not provide reagents that give as accurate a diagnosis as possible. Because of the nature of the disease and the need for accurate results, other reagents must be developed to approach 100 % accuracy and specificity in diagnosis of HTLV-1.

Proteins contain a number of epitopes or antigenic determinants which are the regions of the proteins which comprise the binding sites for specific antibodies. In general, proteins contain between 5 to 10 epitopes, each of which comprises a sequence of 6 to 8 amino acids. Epitopes can be either continuous, in which case the 6 to 8 amino acids are present in linear sequence, or discontinuous, in which the amino acids that form the epitope are brought together by the three dimensional folding of the protein. Even though an epitope constitutes only a relatively few amino acids, its reactivity with an antibody is influenced by the amino acids in the protein which surround the epitope.

Studies aimed at mapping antigenic sites or epitopes of proteins have been aided by the use of synthetic peptides corresponding to various regions of the proteins of interest. See, e.g., Lerner et.al., "The Development of Synthetic Vaccines" in: The Biology of Immunological Disease: A Hospital Practice Book, Dixon and Fisher, eds., pp. 331-338 (1983); Lerner, Adv. Immunol., "Antibodies of Predetermined Specificity in Biology and Medicine" 36:1 (1984). Epitopes may be found in hydrophilic sequences probably corresponding to amino acid residues found on the surface of the protein. Several predictive methods have been advanced based on models of protein folding. Such methods are of limited utility, however, because the antigen-presenting cells of the immune system process proteins to produce epitopes not necessarily corresponding to those found on the protein surface. It is these processed epitopes to which the immune system responds to produce antibodies, therefore it is not possible to predict a priori which peptide seguences represent epitopes actually recognized by the immune system. Peptides representing such processed epitoges should prove extremely useful for diagnostic purposes.

Recombinant peptides, usually about 100 amino acids long, or more, will fold to produce a tertiary structure mimicking that of the intact protein and allowing discontinuous epitopes to form. However, linear internal epitopes might be hidden within the molecule and unaccessible to their specific antibodies.

In addition to their usefulness in epitope mapping studies, synthetic peptides, if encompassing major antigenic determinants of a protein, have potential as immunogenic compositions, including vaccines and diagnostic reagents. Peptides have several advantages in specific antibody production and reactivity. The exact sequence of the peptide can be selected from the amino acid sequence as actually determined by amino acid sequencing of a protein or predicted from the DNA sequence coding for the protein. The use of specific synthetic peptides eliminates the need for using the full-length protein in the production of or assay for specific antibodies. Furthermore, the solid phase peptide synthetic techniques of Merrifield and coworkers allow for essentially unlimited quantities of the synthesized peptide of interest to be chemically produced. See, e.g., Erickson and Merrifield, "Solid Phase Peptide Synthesis", in: The Proteins, 3rd Edit. (1976), Vol 2, Academic Press, New York, Chapter 3. The availability of automated peptide synthesizers has further advanced such techniques.

Although a variety of criteria can be used to determine which regions of proteins are immunodominants, peptides corresponding to such regions may not always be useful in large-scale screening and diagnosis for exsmple, antigenicity may be lost because the peptide is not in a proper spacial orientation which is recognized by antibodies which react with the protein. Furthermore, as is particularly evident with HIV-1 and HIV-2, there is significant genetic variability within each of these two virus groups leading to many serotypes of the viruses. This has put a significant constraint on choosing a region of a protein from which to derive a peptide for use in screening and diagnosis and in formulating vaccines. However, certain immunodominant portions of HIV-1 and HIV-2 proteins have been found to be relatively invariant.

Recently, such immunologically reactive peptides corresponding to various immunodominant regions of the surface glycoproteins gp120 snd gp41 from HIV-1 and the corresponding proteins of HIV-2 encoded by the env gene of the two viruses have been synthesized and shown to react with about 100 % efficiency with sera from HIV-1 or HIV-2 infected individuals. When used in assays for detecting the presence of antibodies, such peptides gave no false positive or false negative reactions.

It is believed that a similar approach for diagnosis of HTLV-1 infection using peptides derived from immunologically important proteins of HTLV-1 would be extremely useful especially in those areas of the world where the virus appears to be endemic.

Several publications have recently presented data showing immunological reactivity of selected synthetic peptides corresponding to antigenic proteins of HTLV-1. In one study several HTLV-1 gag peptides were synthesized. Palker et.al., "C-terminal Region of Human T-cell Lympthotropic virus type I (HTLV-1) p core Protein is Immunogenic in Humans and Contains an HTLV-1 Specific Epitope", J. Immunol. 136:2393-2397 (1986). One of the gag peptides designated SP-71, which corresponds to the C-terminus of the HTLV-1 p19 protein, was found to react with 8/9 HTLV-1 patient sera in a radioimmunoassay (RIA). The amino acid sequence of SP-71 is: Pro-Tyr-Val-Glu-Pro-Thr-Ala-Pro-Gln-Val-Leu. Copeland et.al., "Envelope Proteins of Human T-cell Leukemia Virus Type I: Characterization by Antisera to Synthetic Peptides snd Identification of Natural Epitope", J. Immunol., 137:2945-2951 (1986), synthesized three additional HTLV-1 peptides which correspond to regions of the protein product encoded by the env gene of HTLV-1. One of these peptides, SP-70, which is located near the C terminus of the major surface glycoprotein gp46, had antigenic activity but reacted with only 4/12 sera from HTLV-1 positive patients. Peptide SP-70 is encoded by the nucleotide sequence of the HTLV-1 genome encompassing base pairs 6066-6098 and has the amino acid sequence: Pro-Pro-Phe-Ser-Leu-Ser-Pro-Val-Pro-Thr-Leu-NH₂.

Peptides corresponding to regions of Immunologically important proteins of HTLV-1 such as gp46 which would react with close to 100 % efficiency with sera from HTLV-1 infected patients would find immediate use in diagnostic methods and as potential immunogenic compositions for eliciting the production of antibodies against HTLV-1.

### Description of the drawings

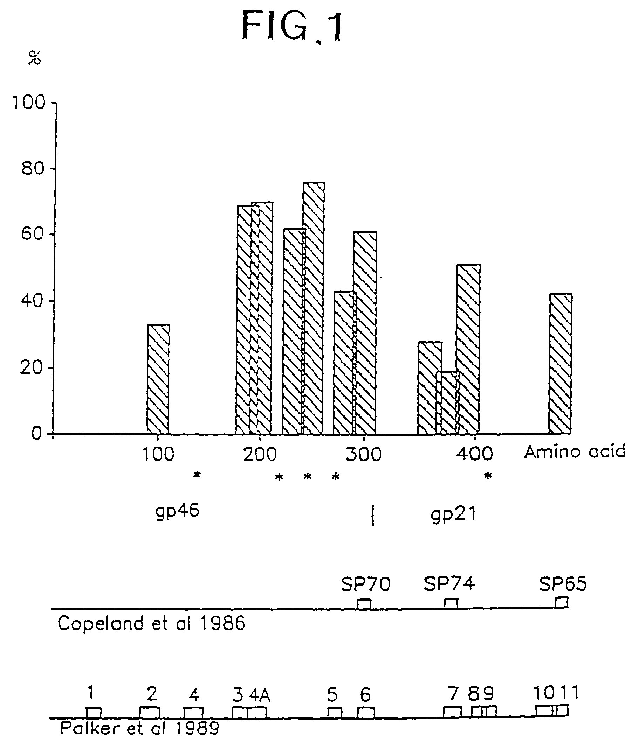

Figure 1 is a bar graph depicting the reactivity of the peptides of the present invention to HTLV positive antisera. The relative position of the peptides along the amino acid sequence of the glycoproteins gp46 and gp21, as well as the percentage of HTLV-1 samples (N=71) reactive to the respective peptide are shown. Potential glycosylation sites are marked with an asterisk.

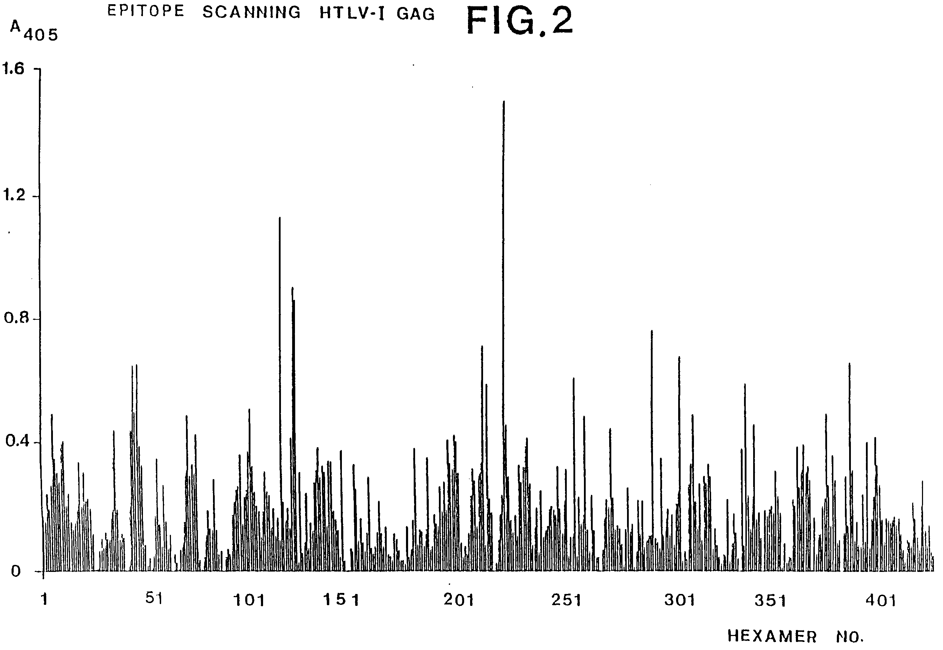

Figure 2 depicts the results of epitope mapping of the HTLV-1 gag gene encoded proteins (gp15, gp19 and gp24). The numbers on the X-axis represent the amino acid number of the first (amino-terminal) amino acid of the respective hexapeptide. The Y-axis shows the corresponding optical density reading obtained for each peptide with two pooled HTLV-1 positive sera.

### Summary of the invention

In accordance with the present invention nine, novel synthetic peptides corresponding to epitopes of HTLV-1 gag and HTLV-1 env proteins are provided. These peptides are useful, alone or in combination, uncoupled or coupled to other molecules, in selective diagnostic methods for detecting HTLV-1 infections, in immunization against HTLV-1 infection and in production of polyclonal and monoclonal antibodies.

### Detailed description of the invention

The novel peptides are useful in tests to diagnose HTLV-1 infection or prior exposure to the virus and as immunogens in compositions to elicit the production in animals including man of antibodies against HTLV-1. The peptides encompassed by the invention comprise oligopeptides having amino acid sequences containing therein sequences which comprise continuous (linear) epitopes reactive with HTLV-1 specific antibodies.

In order to detect peptides corresponding to epitopes, thirtyfive different peptides of 18 to 26 amino acid residues were synthesized. The amino acid sequences of the pegtides are shown in Table 1. These peptides overlapped one another by five or more amino acid residues and covered the entire env gene translational product (twenty-one gp46 peptides and fourteen gp21 peptides). The amino acid sequences were obtained from the predicted amino acid sequence. Seiki et.al. (1983) "Human Adult T-cell Leukemia Virus: Complete Nucleotide Sequence of the Provirus Genome Integrated in Leukemia Cell DNA", Proc. Natl. Acad. Sci. USA, 80:3618-3622. Four of the peptides were obtained previously (A-HTLV-1; B-HTLV-1; C-HTLV-1; and H-HTLV-1), see WO 89/08664.

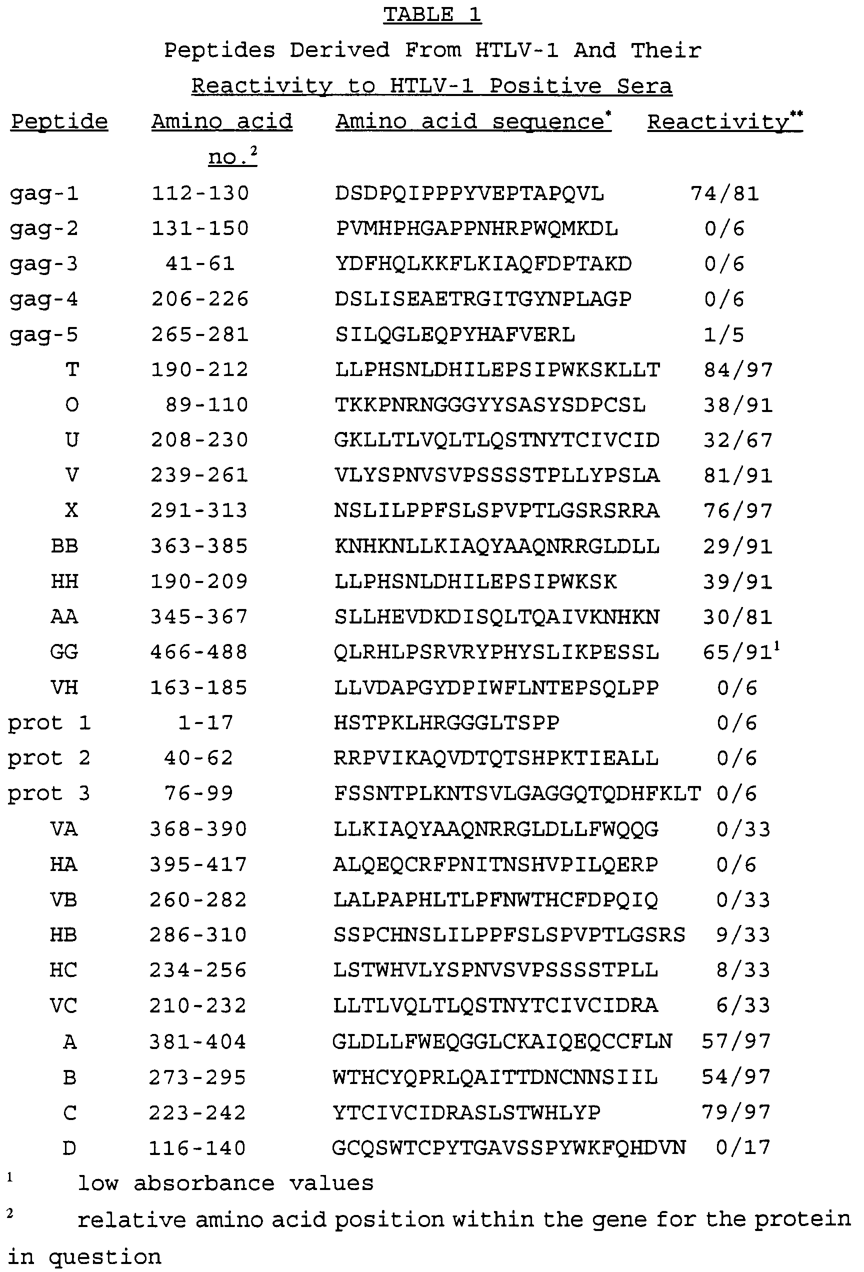

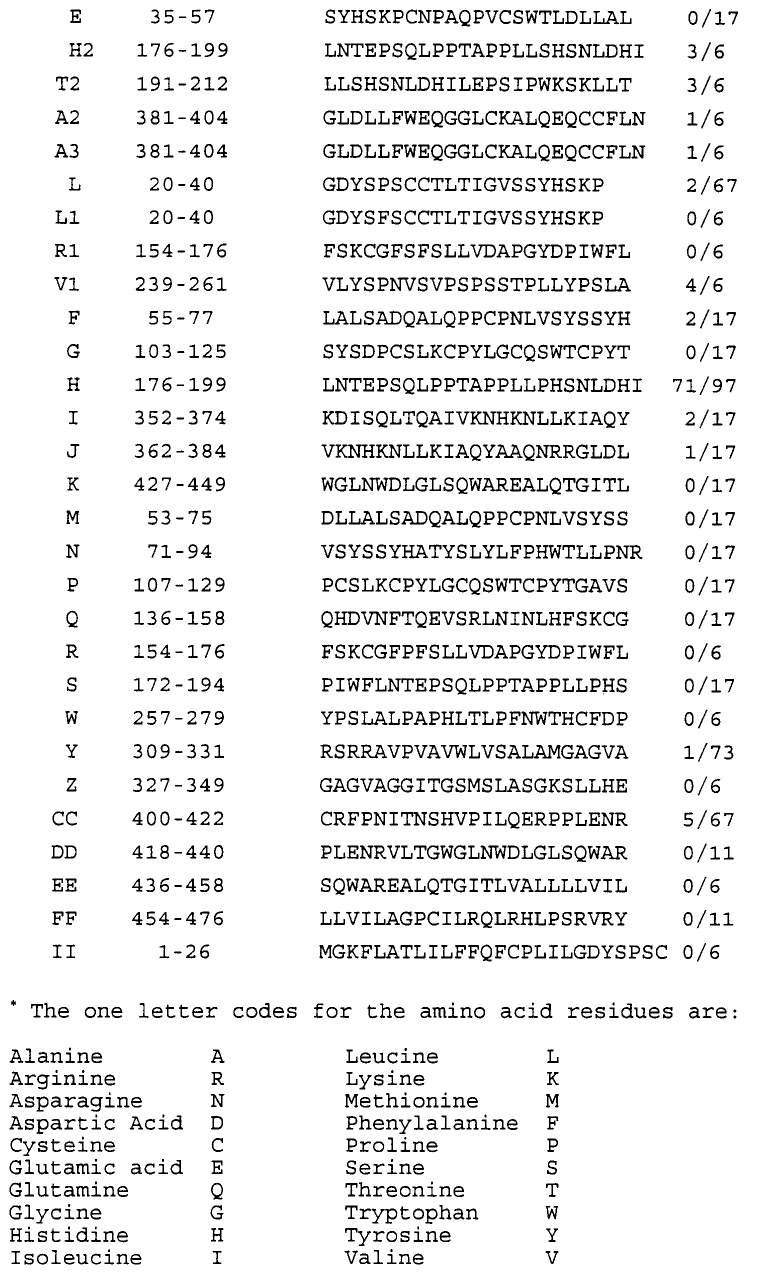

The thirty five different peptides were bound to the wells of microtiter plates and screened for reactivity with six sera obtained from HTLV-1 positive patients using an enzyme linked immunoabsorbent assay (EIA). The eleven peptides that gave a positive reaction with one or more of the serum samples were then further tested with an additional 65 HTLV-1 positive serum samples. The amino acid sequences of the peptides as well as the fraction of HTLV-1 positive sera that reacted with each peptide are shown in Table 1.

The results indicate that there are three highly immunogenic regions located on the env protein: the first is between amino acid residues 176 to 212, defined by peptides H-HTLV-1 (amino acids 176 to 199) and J-HTLV-1 (amino acids 190 to 212); the second is between amino acids 223 to 261, defined by peptides G-HTLV-1 (amino acids 223 to 243) and V-HTLV-1 (amino acids 239 to 261); and the third is between amino acids 273 to 313, defined by peptides B-HTLV-1 (amino acids 273 to 295 ) and X-HTLV-1 amino acids 291 to 313 ). One peptide from the amino-terminal half of gp46 (O-HTLV-1 amino acids 89 to 110) gave a positive reaction with the HTLV-1 positive sera.

Recently a peptide corresponding to amino acid residues 374 to 392 (numbering of gp46/gp21 in consecutive sequence) reacted with 18% of HTLV-1 positive sera. Palker et.al. (1989) "Mapping of Immunogenic Regions of Human T Cell Leukemia Virus type I (HTLV-1) gp45 and gp21 Envelope Glycoproteins with env-encoded Synthetic Peptides and Monoclonal Antibody to gp46" , J. Immunol., 142:971-978. Other researchers have faiied to find peptides corresponding to epitopes within gp21. Copeland et.al. (1986).

Contrary to previous studies indicating that gp21 is not immunogenic, the peptides of the present invention derived from the sequence of gp21 yielded four peptides that had a positive reaction with HTLV-1 positive antisera. The present invention has revealed two regions in gp21 containing epitopes immunogenic in man (Figure 1). One region of the amino-terminal half of gp21 corresponding to amino acids 345-404 was defined by three peptides, AA-HTLVT-1 (amino acids 345- 367), BB-HTLV-1 (amino acids 363 to 385) and A-HTLV-1 (amino acids 381 to 404). The other region containing an epitope was the carboy-terminus of gp21 as defined by peptides GG-HTLV-1 (amino acids 466 to 488). Interestingly, a peptide covering the only potential N-linked glycosylation site in gp21 at amino acids 404 to 406, was not reactive to any of the HTLV-1 positive human sera tested.

The peptides of Figure 1 were synthetized with an Applied Biosystems 430A peptide synthesizer. The peptides were cleaved from the solid phase and the protective side chains were removed with hydrogen fluoride. The peptides were coupled to bovine serum albumin (BSA) with SPDP according to Pharmacia's method (Pharmacia, Uppsala, Sweden). To the original peptide sequence cystein has been added at the C-terminus in order to facilitate the coupling reaction with SPDP. The peptide BSA complex was then loaded onto microtiter plates (NUNC, Denmark). The sera were tested for reactivity against the peptides in 1/50 dilution in indirect ELISA. The sera which gave an absorbance value ≥ mean value of five negative controls + 6SD were considered to give a positive reaction with the peptide. The relative position of the peptide along the amino acids sequence of the glyco-proteins and the percentage HTLV-1 sera (n=71) which reacted with the respective peptide are stated. Only the seven gp46 peptides and the four reactive gp21 peptides that bound to antibodies in HTLV-1 positive area have been stated. Possible glykosylation sites have been marked (*). The localisation of the env peptides tHat have been described by Copeland et.al. (1986) and Palker et.al. (1989) have also been stated.

Another series of peptides were synthesized in order to determine the epitopes of HTLV-1 gag gene products. A different screening strategy was used compared to the one described above. Hexamer peptides overlapping one another by five amino acid residues and covering the entire gag gene product were synthesized on polyethylene rods as described by Geysen et.al. (1984) "Use of Peptide Synthesis to Probe Viral Antigens for Epitopes to a Resolution of a Single Amino Acid", Proc. Natl. Acad. Sci. USA, 81:3998-4002, the frame for the peptide synthesis was moved one amino acid C-terminally for each new peptide (a copy was made for each individual peptide 1). An EIA was then performed with the peptides deprotected but still bound to the rods. The peptides were tested using two pooled sera which had reacted strongly to all three gag encoded proteins. The final dilution of each serum was 1/100. The results obtained are shown in Figure 2. Guided by these results, five peptides (seventeen to twenty-one amino acids in length (were synthesized and coated onto microtiter plates as described above. When HTLV-positive sera were tested against the peptides, one peptide reacted strongly (Gag-1-HTLV-1). The numbers on the x-axis correspond to the amino acid coordinats for the first (N-terminal) amino acid of the respective hexa peptide. The Y-axis shows the absorbance value for each peptide against the pool of HTLV-1 positive sera.

The invention thus encompasses the immunplogically reactive peptides and functionally equivalent variants thereof, which do not significantly affect the antigenic properties of the peptides, corresponding to regions of the envelope glycoprotein encoded by the env gene of HTLV-1.

The peptides were synthesized by known solid phase peptide synthesis techniques. See e.g., Merrifield and Barany, The Petides: Analysis, Synthesis, Biology (1980), vol. 1, Gross and Meinenhofer, eds., Academic Press, New York, Chap. 1. The synthesis also allows for one or two amino acids not corresponding to the original protein sequence to be added to the amino or carboxyl terminus of the peptides. Such extra amino acids are useful for coupling the peptides to each other, to another peptide, to a large carrier protein or to a solid support. Amino acids that are useful for these purposes include but are not limited to tyrosine, lysine, glutamine acid, aspartic acid, cysteine and derivatives thereof. Additional protein modification techniques may be used, e.g., NH₂-acetylation or COOH-terminal amidation, to provide additional means for coupling the peptides to another protein or peptide molecule or to a support.

Also included are analogues and homologs of the peptides. Analogues are peptides which are functionally equivalent to the present peptides but which contain nonnaturally occuring amino acids. Homologs are peptides which have conservatively substituted amino acids or correspond to peptides encoded by the genome of any other isolate of HTLV-1. Conservative amino acid substitutions include but are not limited to: glycine, alanine; valine, isoleucine, leucine; asparagine, glutamine; aspartic acid, glutamine acid; serine, threonine; lysine, arginine and phenylalanine, tyrosine. The peptides encompassed by the invention comprise amino acid sequences each containing at least one continuous (linear) epitope reactive with HTLV-1 antibodies. Thus derivatives of the peptides containing less than the complete amino acid sequences shown below are embodied by the present invention provided they contain at least one linear epitope recognized by antibodies specific to HTLV-1.

The expression, "the peptides" thus includes the amino acid sequences give, analogues, homologs and epitopes derived therefrom.

The novel peptides corresponding to the HTLV-1 proteins are set forth below. The numbering of the amino acid residues has been previously described by Seiki et.al., Proc. Natl. Acad. Sci. USA, 80:3618-3622 (1983). The amino acid abbreviations are: Ala, alanine; Arg, arginine; Asn, asparagine; Asp, aspartic acid; Cys, cysteine; Gln, glutamine; Glu, glutamic acid; Gly, glycine; His, histidine; Ile, isoleucine; Leu, leucine; Lys, lysine; Met, methionine; Phe, phenylalanine; Pro, proline; Ser, serine; Thr, threonine; Trp, tryptophan, Tyr, Tyrosine; Val, valine.

### Peptide I-HTLV-1

X-Lys-Asp-Ile-SerGln-Leu-Thr-Gln-Ala-Ile-Val-Lys-Asn-His-Lys-Asn-Leu-Leu-Lys-Ile-Ala-Gln-Tyr-Y-X,
analogues and homologs thereof, wherein X is either a hydrogen molecule of the amino terminal NH₂ group of the peptide or an additional amino acid bonded to the amino terminal NH₂ group of the peptide, the additional amino acid being selected to facilitate coupling of the peptide to peptides, proteins or other support; Y is absent or a cysteine residue; and Z is a hydroxyl group or amino group.

Peptide I-HTLV-1 is derived from the env protein of HTLV-1. Peptide I-HTLV-1 in which X is hydrogen, Y is a cysteine residue and Z is a hydroxyl group is particularly preferred.

### Peptide AA-HTLV-1

X-Ser-Leu-Leu-His-Glu-Val-Asp-Lys-Asp-Ile-Ser-Gln-Leu-Thr-Gln-Ala-Ile-Val-Lys-Asn-His-Lys-Asn-Y-Z, analogues and homologs thereof, wherein X, Y and Z have the same definition as above.

Peptide AA-HTLV-1 is derived from the env protein of HTLV-1. Peptide AA-HTLV-1 in which X is hydrogen, Y is a cysteine residue and Z is a hydroxyl group is particularly preferred.

### Peptide BB-HTLV-1

X-Lys-Asn-His-Lys-Asn-Leu-Leu-Lys-Ila-Ala-Gln-Tyr-Ala-Ala-Gln-Asn-Arg-Arg-Gly-Leu-Asp-Leu-Leu-Y-Z,
analogues and homologs thereof, wherein X, Y and Z have the same definition as above.

Peptide BB-HTLV-1 is derived from the env protein of HTLV-1. Peptide BB-HTLV-1 in which X is hydrogen, Y is a cysteine residue and Z is a hydroxyl group is particularly preferred.

### Peptide GG-HTLV-1

X-Gln-Leu-Arg-His-Leu-Pro-Ser-Arg-Val-Arg-Tyr-Pro-His-Tyr-Ser-Leu-Ile-Lys-Pro-Glu-Ser-Ser-Leu-Y-Z,
analogues and homologs thereof, wherein X, Y and Z have the same definition as above. Peptide GG-HTLV-1 is derived from the env protein of HTLV-1. Peptide GG-HTLV-1 in which X is hydrogen, Y is a cysteine residue and Z is a hydroxyl group is particularly preferred.

### Peptide O-HTLV-1

X-Thr-Lys-Lys-Pro-Asn-Arg-Asn-Gly-Gly-Gly-Tyr-Tyr-Ser-Ala-Ser-Tyr-Ser-Asp-Pro-Cys-Ser-Leu-Y-Z,
analogues and homologs thereof, wherein X, Y and Z have the same definition as above. Peptide O-HTLV-1 is derived from the env protein of HTLV-1. Peptide 0-HTLV-1 in which X is hydrogen, Y is a cysteine residue and Z is a hydrogyl group is particularly preferred.

### Peptide T-HTLV-1

X-Leu-Leu-Pro-His-Ser-Asn-Leu-Asp-His-Ile-Leu-Glu-Pro-Ser-Ile-Pro-Trp-Lys-Ser-Lys-Leu-Leu-Thr-Y-Z,
analogues and homologs thereof, wherein, X, Y and Z have the same definition as above. Peptide T-HTLV-1 is derived from the env protein of HTLV-1. Peptide T-HTLV-1 in which X is hydrogen, Y is a cysteine residue and Z is a hydroxyl group is particularly preferred.

### Peptide V-HTLV-1

X-Val-Leu-Tyr-Ser-Pro-Asn-Val-Ser-Val-Pro-Ser-Ser-Ser-Ser-Thr-Pro-Leu-Leu-Tyr-Pro-Ser-Leu-Ala-X-Z,
analogues and homologs thereof, wherein X, Y and Z have the same definition as above. Peptide V-HTLV-1 is derived from the env protein of HTLV-1. Peptide V-HTLV-1 in which X is hydrogen, Y is a cysteine residue and Z is a hydroxyl group is particularly preferred.

### Peptide X-HTLV-1

X-Asn-Ser-Leu-Ile-Leu-Pro-Pro-Phe-Ser-Leu-Ser-Pro-Val-Pro-Thr-Leu-Gly-Ser-Arg-Ser-Arg-Arg-Ala-Y-Z,
analogues and homologs thereof, wherein X, Y and Z have the same definition as above. Peptide X-HTLV-1 is derived from the env protein of HTLV-1. Peptide X-HTLV-1 in which X is hydrogen, Y is a cysteine residue and Z is a hydroxyl group is particularly preferred.

### Peptide GAG-1-HTLV-1

X-Asp-Ser-Asp-Pro-Gln-Ile-Pro-Pro-Pro-Tyr-Val-Glu-Pro-Thr-Ala-Pro-Gln-Val-Leu-Y-Z,
analogues and homologs thereof, wherein X, y snd Z have the same definition as above. Peptide GAG-1-HTLV-1 is derived from the gag protein of HTLV-1. Peptide GAG-1-HTLV-1 in which R is hydrogen, Y is a cysteine residue and Z is a hydroxyl group is particularly preferred.

The peptides can be used alone or in combination in methods for detection of antibodies to HTLV-1 or HTLV-1 associated antigens. Antibodies can be found in biological samples including but not limited to sera, other body fluids, tissue samples and other samples which may contain antibodies to HTLV-1. Particularly H-HTLV-1, 4-HTLV-1 and T-HTLV-1 can be used to distinguish HTLV-1 infection from HTLV-2 infection. The invention is useful for screening blood and blood-derived products with a high degree of reliability and specificity. The peptides are also useful as vaccines to protect against future infection by HTLV-1. The invention also provides monoclonal and polyclonal antibodies which specifically recognize the peptides.

The methods which use the peptides to detect the presence of HTLV-1 specific antibodies in the sample involve contacting the sample with at least one of the peptides under conditions which allow the formation of an immunological complex between the peptide and any antibodies to HTLV-1 that may be present in the sample. The formation of an immunological complex, if any, indicating the presence of antibodies to HTLV-1 in the sample, is then detected and measured by suitable means.

Such detection methods include but are not limited to homogeneous and heterogeneous binding immunoassays, such as radioimmunoassays (RIA), ELISA and Western blot analyses. Further, the assay protocols using the novel peptides allow for competitive and non-competitive binding studies to be performed. The screening methods are rapid, efficient and allow for simultaneous screening of numerous samples.

The peptides may be labeled (signal-generating) or unlabeled depending on the type of assay used. Labels which may be coupled to the peptides are those known is the art and include but are not limited to enzymes, radionuclides, fluorogenic and chromogenic substrates, cofactors, biotin/avidin, colloidal gold, and magnetic particles.

The peptides can be coupled by any means known in the art to other peptides, solid supports and carrier proteins. Such solid supports include but are not limited to polystyrene or polyvinyl microtiter plates, glass tubes or glass beads and chromatographic supports, such as paper, cellulose and cellulose derivates, and silica. Carrier proteins include but are not limited to bovine serum albumin (BSA) and keyhole limpet hemocyanin (KLH).

Preferred assay techniques, especially for largescale clinical screening of patient sera and blood and blood-derived products are ELISA, agglutination and Western blot techniques, ELISA tests being particularly preferred for speed, the ability to test numerous samples simultaneously and ease of automation. The ELISA tests employing the peptides described above are based on those currently in use for detecting other viruses. For use as reagents in these assays, the peptides of the invention are conveniently bonded to the inside surface of microtiter wells. The peptides may be directly bonded by hydrophobic interactions to the microtiter well, or attached covalently by means known in the art to a carrier protein, such as BSA, with the resulting conjugate being used to coat the wells. Generally the peptides were used in a concentration of approximately between 1 to 100 µ/ml although this range is not limiting, for instance as much as 500 p/ml of a peptide may be required for the assay to be successful. Generally the peptides are used in concentration of between 10 to 100 µ/ml for coating.

Samples including but not limited to body fluids and tissue samples, are then added to the peptide coated wells, where an immunological complex forms if antibodies to HTLV-1 are present in the sampls. A signal generating means may be added to aid detection of complex formation. A detectable signal is produced if HTLV-1 specific antibodies are present in the sample. Agglutination assays are commonly used in Japan. Either latex or erythrocytes can be used in the technique. The methods used in agglutination assays are well known in the art of blood screens.

The peptides of the invention may also be formulated into compositions for use as immunogens. These immunogenes can be used as or to elicit production of antibodies in animals and man against HTLV-1. The antibodies produced can be either polyclonal or monoclonal. For formulation of such compositions, an immunogenically effective amount ot at least one of the peptides is admixed with a physiologically acceptable carrier suitable for administration to animals including man. The peptides may be covalently attached to each other, to other peptides, to a protein carrier or to other carriers, incorporated into liposomes or other such vesicles, or complexed with an adjuvant or adsorbent as is known in the vaccine art. Alternatively, the peptides are not complexed with the above and merely admixed with a physiologically acceptable carrier such as normal saline or a buffering compound suitable for administration to animals including man. Methods of vaccine and antibody production, purification and characterization are known in the art and will not be described in detail.

As with all immunogenic compositions for eliciting antibodies, the immunogenically effective amounts of the peptides of the invention must be determined empirically. Factors to be considered include the immunogenicity of the native peptide, wether or not the peptide will be complexed with or covalently attached to an adjuvant or carrier protein or other carrier and route of administration for the composition, i.e. intravenous, intramuscular, subcutaneous, etc., and number of immunizings to be administered. Such factors are known in the vaccine art and it is well within the skill of immunologists to make such determinations without undue experimentation.

The invention also encompasses antibodies made in response to the peptide and which recognize the peptides. Such antibodies can be either polyclonal or monoclonal. Methods for making antibodies are well known in the art.

The invention is further illustrated by the following specific examples which are not intended in any way to limit the scope of the invention.

### Example 1

An Applied Biosystems peptide-synthesizer Model 430 A, was utilized for the synthesis of all of the peptides. Each synthesis used a p-methylbenzylhydrylamine solid phase support resin (Peptides International, Louisville. KY). The peptides were synthesized according to the Users Manual for Peptide Synthesizer Model 430A, Applied Biosystems, 1986.

All amino acids for use in synthesis contained t-butylcarbonyl groups (t-Boc) protecting the α-NH₂ group and were obtained from Novabiochem AG, Switzerland. Amino acids with reactive side chain groups contained additional protective groups to prevent unwanted and undesirable side chain reactions. The individual protected amino acids used in synthesizing all of the peptides are set forth in Table 2.

After completion of a particular synthesis, the protecting groups were removed from the synthesized peptide and the peptide was cleaved from the solid support resin by treatment at 0°C with anhydrous hydroflouric acid (HF) combining 10 % anisole and 10 % dimethylsulfide as scavenging agents. After cleavage, the HF in the sample was purged under a stream of N₂, with removal of any residual HF accomplished by subjecting the sample to vacuum at 0°C. The peptides were extracted from the resin by treatment with trifluouroacetic acid (TFA) which was then removed by evaporation at room temperature. Following TFA removal, the peptides were precipitated and washed with anhydrous ether.

Prior to use in specific assays, the peptides can be further purified, if desired, by reverse phase high performance liquid chromatography (HPLC). A particularly suited column for such purification is the reverse-phase Vydak ^{R} C-18 column using a water (TFA) - acetonitrile (TFA) gradient to elute the peptides.

**Table 2**

| Amino Acids Used in the Synthesis of Peptides |
|---|
| Boc-Ala-OH |
| Boc-Arg (Tos)-OH |
| Boc-Asn-OH |
| Boc-Asp (OBzl)-OH |
| Boc-Cys (pMeOBzl)-OH |
| Boc-Glu (OBzl)-OH |
| Boc-Gln-OH |
| Boc-Gly-OH |
| Boc-His (Tos)-OH |
| Boc-Ile-OH 1/2 H₂O |
| Boc-Leu-OH H₂O |
| Boc-Lys (2-CI-Z)-OH (cryst.) |
| Boc-Met-OH |
| Boc-Phe-OH |
| Boc-Pro-OH |
| Boc-Ser (Bzl)-OH DCHA |
| Boc-Thr (Bzl)-OH |
| Boc-Trp (Formyl)-OH |
| Boc-Tyr (2-Br-Z)-OH |
| Boc-Val-OH |
| Tos = Tosyl or p-Toluene sulfonic acid oBzl = Benzyloxy pMeoBzl = p-Methylbenzyloxy 2-Cl-Z = Carbobenzoxy chloride 2-Br-Z = Carbobenzoxy bromide |

### Example 2

### Preparation of Microtiter Plates for ELISA Assay

The following experiments were performed by using peptides conjugated to Bovine serum Albumin (BSA) to facilitate coating of the wells of the microtiter plates.

For production of 200 peptide coated microtiter plates the following protocol is used.

An aliquot of 0.15 g BSA (Boehringer Mannheim, fraction V) is dissolved in 3 ml coupling buffer (0.2M Na P0₄, PH 8.5). This BSA solution is divided into three equal volumes each of which is applied to PD-10 column (Pharmacia AB, Uppsala Sweden) followed by 1.5 ml coupling buffer and eluted with 2.0 ml coupling buffer. The BSA concentration of the pooled eluate samples is calculated by measuring the absorbance of the solution at 280nm, where A₂₈₀ (0.1 % BSA) = 0.67 ml/mg. The recovery is generally 80-90 %.

N-succinimidyl 3-(2-pyridyldithio) propionate (SPDP, Pharmacia) is then dissolved in ethanol to a final concentration of 5-40mM. The concentration of SPDP is determined by measuring the reactive ester according to the Pharmacia Fine Chemicals SPDP brochure. 2-pyridyldisulfide residues are introduced into the BSA prepared as above by adding ten SPDP equivalents to each BSA equivalent. The SPDP solution is introduced to the BSA solution with stirring. The mixture is then incubated for 15-30 minutes at room temperature.

To remove excess unreacted SPDP, the pyridyldisulfide-BSA mixture is aliquoted into 6 equal volumes each of which is applied to a PD-10 column. The columns are equilibrated and the product is eluted with 10 % acetic acid in water. The degree of substitution is measured according to the Pharmacia Fine Chemicals SPDP brochure. The recovery of BSA is generally 90-120 mg and the degree of substitution is approximately 7 with a range of 6-8.

The peptide solution is made by mixing 25 mg peptide and an amount of the pyridyldisulfide-BSA solution to make seven peptide equivalents to one BSA equivalent. The mixture is incubated for 18-48 hours at room temperature. The released 2-thiopyridone is removed by running the reaction mixture over a column (2.0cm², 80-100 ml) packed with Sephadex G-25 (Pharmacia-LKB), equilibrated with 10 % acetic acid in water. The product is eluted with 10 % acetic acid in water. The fractions with A₂₈₀ greater than 0.5 are then collected and pooled. The pooled volume is approximately 30-40 ml. The A₂₈₀ of the pooled fractions is measured and the BSA concentration is calculated. The pooled fractions are stored at 4° C and are stable for months.

The peptide-BSA conjugate is then diluted in coating buffer (50mM NaCO₃, 0.15M NaCl, pH 9.5) to 60 mg/ml. The pH of the solution is checked and adjusted with 1-5M NaOH, to 9.5.

An aliguot of 100 µl of the peptide-BSA mixture in coating buffer is placed in each well of microtiter plate (Nunc, High Binding, catalog No. 4-68667). The plates are incubated for 15 minutes at room temperature. After the incubation, the liquid is aspirated from the wells. An aliquot of 200 µl sterile, (0.22µm-filtered) 3 % BSA in phosphate buffered saline (PBS, 1OmM NaPO₄, 0.15M NaCl, PH 7.2) is added to each well. The plates are covered and incubated for sixteen hours at 37° C.

After the incubation, the liquid is aspirated from the wells. The microtiter plates are glaced in a safety cabinet and air dried for about three hours.

The plates can be stored for a long time st +4° C or -20° C in any closed container, for instance on sealed aluminium bags. The presence of a drying agent such as silica gel aids in preservation of the plates.

### Example 3

### ELISA Methods

The peptides were used in an ELISA test to measure their immunologic reactivity. All peptides were run in parallel ELISA tests against serum samples positive for antibodies to HTLV-2, serum samples positive for antibodies to HTLV-1 and 10 blood donor sera negative for HTLV-1/HTLV-2. The sera were also tested against HTLV-1 peptides which have been previously described in PCT patent publication, W089-08664.

The microtiter plates are prepared as in Example 2. If the plates have been stored they may first be brought to room temperature and then they may be pre-soaked for ten minutes in wash buffer (0.05 % Tween 20 in PBS). The presoak solution is then aspirated from the wells prior to use.

The serum samples are each diluted 1:50 in serum dilution solution (1 % HSA in wash buffer). An aliquot of 100 µl diluted serum is placed in each well and the plates are incubated for 90 minutes at 37° C in a humidifier. After the incubation, the plates are washed three times with wash buffer.

Anti-human immunoglobulin G (IgG) conjugate (Jackson, from Labassco, art. nr. 10.4999999, 109-056-003, Alkaline Phosphatase) is dissolved in 0.5 ml H₂0, aliquoted and frozen. Frozen aliquots are thawed and diluted 1:5000 in serum dilution buffer. An aliquot of 100 µl is added to each well. The plates are incubated for 90 minutes at 37° C in a humidified chamber. After the incubation, the plates are washed three times with wash buffer.

Alkaline phosphatase substrate (Sigma, tablets) is dissolved in substrate dilution buffer (50mM Na₂CO₃, 1mM MgCl₂) to a final concentration of 1 mg/ml. An aliquot of 200 µl is added to each well. The plates are incubated for approximately 35 minutes at room temperature. If desired the reaction can be stopped by the addition of 100 µl 3M NaOH per well.

To determine the amount of antibody bound to the peptides in each well, the plates are read at 405 nm. The higher the absorbance, the greater the amount of bound antibody.

### Example 4

### ELISA Test of Initial Peptides with HTLV-2 and HTLV-1 Positive Sera

Peptides derived from HTLV-2, homologous to A-HTLV-1; AA-HTLV-1, B-HTLV-1, C-HTLV-1, HH-HTLV-1, V-HTLV-1, and X-HTLV-1 were made by the method described in Example 1. The HTLV-2 peptides are described in U.S. Patent Application Serial No. 07/434,239, filed Nov. 13, 1989. The HTLV-2 amino acid sequences were derived from the nucleotide seguence described by Shimitohno (1985) "Complete Nucleotide Sequence of an Infectious Clone of Human T cell Leukemia Yirus Type II: An Open Reading Frame for the Protease", Proc. Nat'l. Acad. Sci. U.S.A. 83:3101-3015. This group of peptides homologous peptides was tested with patient sera by the method described in Examples 2 and 3 to determine determine the ability of the particular peptides to detect antibodies which recognize HTLV-2, distinguish between HTLV-1 and HTLY-2 and are crossreactive.

The serum samples used in this screening test were confirmed HTLV-2 positive by PCR analysis, The sera designated HT-201-HT-220 were obtained from Serologicals Inc., Pensacola, Fla. Previous researchers were unable to distinguish between antibodies specific for either HTLV-1 or HTLV-2 in these sera by any of Western blot analyses, ELISA tests and immunofluorescense assays.

Table 3 shows the results obtained by the ELISA test. The negative controls are sera negative for both HTLV-1 and HTLV-2 and are designated NC-1 and NC-2, the HTLV-1 positive serum is designated HTLV-1. The patient sera HT-201-HT-220 are designated 201-220. The results presented are absorbance readings at 405nm.

The results presented in Table 3 clearly show that H-HTLV-2, O-HTLV-2, T-HTLV-2 and Gag-1-HTLV-2 react strongly with antibodies present in HTLV-2 infected patient sera. All of the peptides react poorly with the sera designated HT-218 and HT-219. These sera were found to be only weakly positive with previous ELISA tests indicating that the level of HTLV2 specific antibodies in these sera was low. Surprisingly, the HTLV-2 peptides react well with HTLV-2 infected patient sera and poorly with antibodies present in HTLV-1 infected patient sera. The Gag-1-HTLV-2 peptide is not as specific as the other three peptides.

It is surprising that the remaining peptides were unable to detect antibodies to HTLV-2 in the majority of patient sera. These peptides correspond to purported HTLV-1 epitopes snd were therefore expected to react well with HTLV-2 antibodies.

### Example 5

### Specificity of the Peptides

In order to better define the specificity of the HTLV-1 peptides, ELISA tests were done as described in Example 3. Peptides H-HTLV-1, O-HTLV-1, and T-HTLV-1, were compared to H-HTLV-2, O-HTLV-2, and T-HTLV-2 by testing against both HTLV-1 and HTLV-2 positive sera. The patient sera were obtained from Dr William Hall, Cornell University, N.Y.

Table 4 shows the results obtained. The sera designated 2a, 2b, 2c, 2d, 2e and 2f were obtained from five different patients and were HTLV-2 positive as determined by polymerase chain reaction (PCR) analysis and were also human immunodeficiency virus (HIV) positive. The sera designated 1a, 1b, 1c, 1d, 1e and 1f were obtained from five different patients and were HTLV-1 positive as determined by PCR analysis. Patients 1a, 1b and 1c have adult T cell leukemia and patients 1d, 1e and 1f are HIV positive. Sera designated HIV-1 were obtained from patients who are neither HTLV-1 nor HTLV-2 positive but are HIV positive. Sera designated NL-1 and NL-2 are negative controls obtained from patients not infected with either HTLV-1 or HTLV-2.

The numbers shown in Table 4 are the average of two experiments and are the absorbance readings at 405nm.

The results obtained illustrate the high degree of specificity obtained by the peptides.

It is evident from the foregoing results that the novel synthetic peptides, described herein, which correspond to regions of proteins encoded by the env and gag genes of HTLV-1, clearly provide unique reagents for a sensitive assays for the presence of antibodies to HTLV-1. Also, peptides H-HTLV-2 clearly discriminate between antibodies which recognize HTLV-1 and antibodies which recognize HTLV-2.

It may in some cases be appropriate to use at least two peptides in assays, since two geptides provide a higher frequency of sero-positivity or sensitivity as compared to one peptide. This also means that the frequency of false negative reactions decreases.

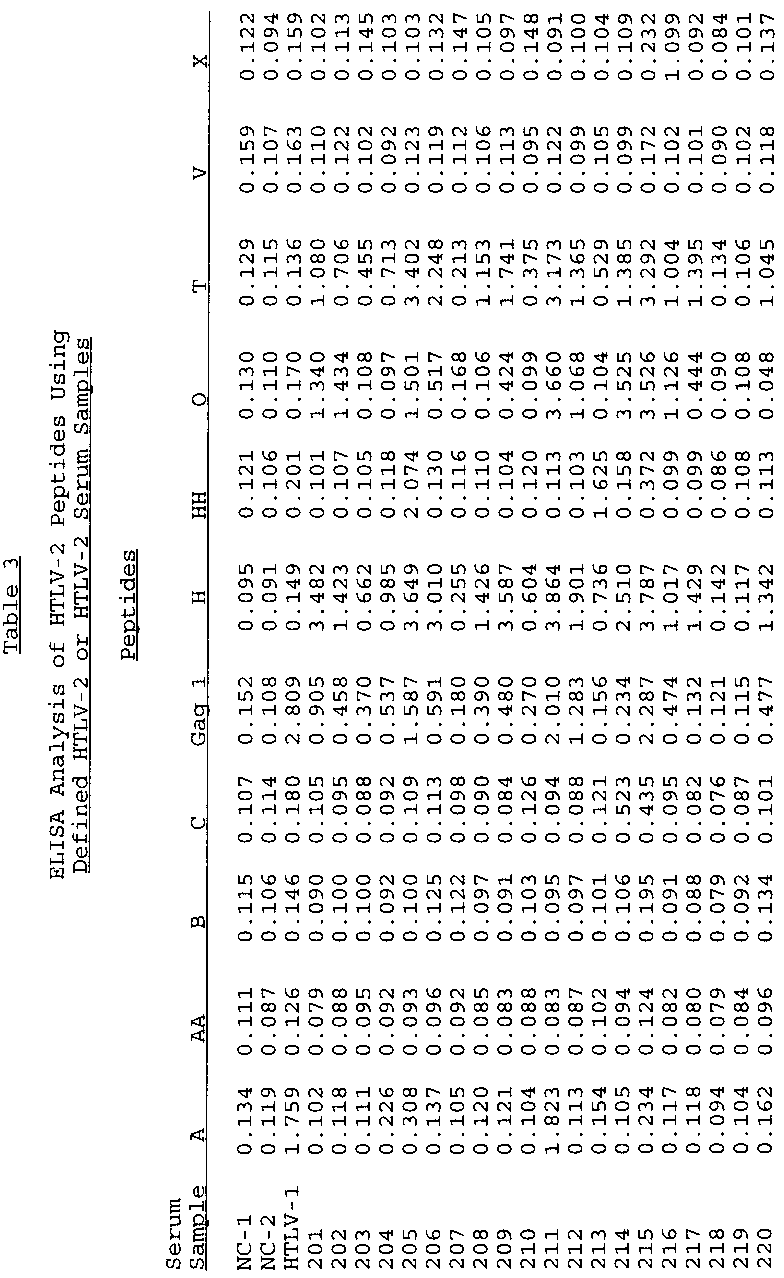

**Tabelle 4**

| ELISA Results of Peptides Derived From HTLV-1 and HTLV-2 From Corresponding Regions Peptide | | | | | | |
|---|---|---|---|---|---|---|
| Patient | H-HTLV-1 | H-HTLV-2 | T-HTLV-1 | T-HTLV-2 | O-HTLV-1 | O-HTLV-2 |
| 2a | 0.016 | 1.162 | 0.026 | 0.908 | 0.055 | 1.753 |
| 2b | 0.041 | 1.441 | 0.068 | 1.228 | 0.043 | 1.000 |
| 2c | 0.051 | 1.803 | 0.078 | 1.476 | 0.040 | 0.846 |
| 2d | 0.028 | 0.925 | 0.054 | 0.774 | 0.049 | 1.338 |
| 2e | 0.039 | 1.371 | 0.043 | 1.729 | 0.069 | 0.648 |
| 2f | 0.023 | 1.788 | 0.048 | 1.644 | 0.060 | 0.757 |
| 1a | 1.889 | 0.044 | 1.735 | 0.072 | 1.742 | 0.072 |
| 1b | 1.963 | 0.091 | 1.773 | 0.100 | 1.963 | 0.076 |
| 1c | 1.865 | 0.027 | 1.838 | 0.130 | 1.878 | 0.096 |
| 1d | 1.938 | 0.062 | 1.830 | 0.109 | 1.508 | 0.098 |
| 1e | 1.923 | 0.065 | 1.799 | 0.043 | 1.466 | 0.124 |
| 1f | 1.872 | 0.044 | 1.912 | 0.076 | 1.757 | 0.088 |
| HIV-1 | 0.079 | 0.089 | 0.053 | 0.130 | 0.100 | 0.127 |
| HIV-1 | 0.061 | 0.052 | 0.096 | 0.110 | 0.110 | 0.112 |
| NL-1 | 0.033 | 0.101 | 0.098 | 0.105 | 0.123 | 0.132 |
| NL-2 | 0.028 | 0.078 | 0.092 | 0.107 | 0.116 | 0.126 |
| NO Serum | 0.020 | 0.082 | 0.052 | 0.095 | 0.049 | 0.083 |

## Claims

1. A peptide recognized by antibodies specific to HTLV-1,
**characterized in that,**
it is selected from a group of peptides having the following amino acid sequences:
a) Leu-Leu-Pro-His-Ser-Asn-Leu-Asp-His-Ile-Leu-Glu-Pro-Ser-Ile-Pro-Trp-Lys-Ser-Lys-Leu-Leu-Thr,
b) Lys-Asn-His-Lys-Asn-Leu-Leu-Lys-Ile-Ala-Gln-Tyr-Ala-Ala-Gln-Asn-Arg-Arg-Gly-Leu-Asp-Leu-Leu,
c) Gly-Leu-Asp-Leu-Leu-Phe-Trp-Glu-Gln-Gly-Gly-Leu-Cys-Lys-Ala-Ile-Gln-Glu-Gln-Cys-Cys-Phe-Leu-Asn,
d) Leu-Asn-Thr-Glu-Pro-Ser-Gln-Leu-Pro-Pro-Thr-Ala-Pro-Pro-Leu-Leu-Ser-His-Ser-Asn-Leu-Asp-His-Ile,
e) Leu-Leu-Ser-His-Ser-Asn-Leu-Asp-His-Ile-Leu-Glu-Pro-Ser-Ile-Pro-Trp-Lys-Ser-Lys-Leu-Leu-Thr,
f) Gly-Leu-Asp-Leu-Leu-Phe-Trp-Glu-Gln-Gly-Gly-Leu-Cys-Lys-Ala-Leu-Gln-Glu-Gln-Cys-Cys-Phe-Leu-Asn,
g) Gly-Leu-Asp-Leu-Leu-Phe-Trp-Glu-Gln-Gly-Gly-Leu-Cys-Lys-Ala-Leu-Gln-Glu-Gln-Cys-Tyr-Phe-Leu-Asn,
h) Val-Lys-Asn-His-Lys-Asn-Leu-Leu-Lys-Ile-Ala-Gln-Tyr-Ala-Ala-Gln-Asn-Arg-Arg-Gly-Leu-Asp-Leu, or
i) Pro-Ile-Trp-Phe-Leu-Asn-Thr-Glu-Pro-Ser-Gln-Leu-Pro-Pro-Thr-Ala-Pro-Pro-Leu-Leu-Pro-His-Ser.

2. A peptide according to claim 1,
**characterized in that**
the peptide has an amino terminus comprising an additional amino acid selected to facilitate coupling of the peptide to a carrier; and the peptide has a carboxy terminus selected from the group consisting of an amino group, an hydroxy group, a cysteine residue, a cysteine residue followed by an amino group and a cysteine residue followed by an hydroxy group.

3. A method for detecting antibodies to HTLV-1 in a biological sample, characterized by contacting the sample with at least one peptide of claim 1 under conditions such that an immuno-logical complex will form between the peptides and antibodies to HTLV-1 if such antibodies are present in the sample; and detecting the formation, if any, of the immunological complex to determine the presence of antibodies to HTLV-1 in the sample.

4. A method according to claim 3,
**characterized in that,**
the peptide has an amino terminus comprising an additional amino acid selected to facilitate coupling of the peptide to a carrier; and the peptide has a carboxy terminus selected from the group consisting of an amino group, an hydroxy group, a cysteine residue, a cysteine residue followed by an amino group and a cysteine residue followed by an hydroxy group.

5. A method for detecting antibodies to HTLV-1 in a sample,
characterized by
contacting the sample with at least two peptides of claim 1, under conditions such that an immunological complex will form between the peptides and antibodies to HTLV-1 if such antibodies are present in the sample and;
measuring the formation, if any, of the immunological complex to determine the presence of antibodies to HTLV-1 in the sample.

6. A method according to any one of claims 3 through 5, wherein said immunological complex formed with an HTLV-1 antibody is referred to as a first complex, further characterized by the steps of
contacting the sample with at least one peptide having at least one epitope recognized by antibodies specific to HTLV-2, such that a second immunological complex will form between such peptides and antibodies to HTLV-2 if such antibodies are present in the sample; and
comparing the formation of said first and second immunological complexes to determine whether antibodies present in the sample were HTLV-1 antibodies or HTLV-2 antibodies.

7. A composition, characterized by at least one peptide selected from a group of peptides having the following amino acid sequences:
a) Leu-Leu-Pro-His-Ser-Asn-Leu-Asp-His-Ile-Leu-Glu-Pro-Ser-Ile-Pro-Trp-Lys-Ser-Lys-Leu-Leu-Thr,
b) Lys-Asn-His-Lys-Asn-Leu-Leu-Lys-Ile-Ala-Gln-Tyr-Ala-Ala-Gln-Asn-Arg-Arg-Gly-Leu-Asp-Leu-Leu,
c) Gly-Leu-Asp-Leu-Leu-Phe-Trp-Glu-Gln-Gly-Gly-Leu-Cys-Lys-Ala-Ile-Gln-Glu-Gln-Cys-Cys-Phe-Leu-Asn,
d) Leu-Asn-Thr-Glu-Pro-Ser-Gln-Leu-Pro-Pro-Thr-Ala-Pro-Pro-Leu-Leu-Ser-His-Ser-Asn-Leu-Asp-His-Ile,
e) Leu-Leu-Ser-His-Ser-Asn-Leu-Asp-His-Ile-Leu-Glu-Pro-Ser-Ile-Pro-Trp-Lys-Ser-Lys-Leu-Leu-Thr,
f) Gly-Leu-Asp-Leu-Leu-Phe-Trp-Glu-Gln-Gly-Gly-Leu-Cys-Lys-Ala-Leu-Gln-Glu-Gln-Cys-Cys-Phe-Leu-Asn,
g) Gly-Leu-Asp-Leu-Leu-Phe-Trp-Glu-Gln-Gly-Gly-Leu-Cys-Lys-Ala-Leu-Gln-Glu-Gln-Cys-Tyr-Phe-Leu-Asn,
h) Val-Lys-Asn-His-Lys-Asn-Leu-Leu-Lys-Ile-Ala-Gln-Tyr-Ala-Ala-Gln-Asn-Arg-Arg-Gly-Leu-Asp-Leu, or
i) Pro-Ile-Trp-Phe-Leu-Asn-Thr-Glu-Pro-Ser-Gln-Leu-Pro-Pro-Thr-Ala-Pro-Pro-Leu-Leu-Pro-His-Ser
and a physiologically acceptable carrier therefor.

8. A composition according to claim 6,
**characterized in that**
the peptide has an amino terminus comprising an additional amino acid selected to facilitate coupling of the peptide to a carrier; and the peptide has a carboxy terminus selected from the group consisting of an amino group, an hydroxy group, a cysteine residue, a cysteine residue followed by an amino group, and a cysteine residue followed by an hydroxy group.

9. A composition, characterized by at least two peptides of claim 1, and a physiologically acceptable carrier therefor.


SEQUENCE LISTING

(1) GENERAL INFORMATION:

(i) APPLICANT:
(A) NAME: Syntello INC.
(B) STREET: 4350 Executive Drive, Suite 310
(C) CITY: San Diego
(D) STATE: California
(E) COUNTRY: U.S.A
(F) POSTAL CODE (ZIP): CA 92121

(ii) TITLE OF INVENTION: Peptides and antibodies derived therefrom for
the diagnosis of, therapy for and vaccination against
HTLV-1 infection

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: SE 8902148-9
(B) FILING DATE: 13-JUN-1989

(vi) PRIOR APPLICATION DATA:
(A) APPLICATION NUMBER: PCT/SE90/00407
(B) FILING DATE: 12-JUN-1990

(2) INFORMATION FOR SEQ ID NO: 1:

(i) SEQUENCE CHARACTERISTICS:
(A) LENGTH: 23 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 1:

Leu Leu Pro His Ser Asn Leu Asp His Ile Leu Glu Pro Ser Ile Pro
1               5                   10                  15

Trp Lys Ser Lys Leu Leu Thr
            20

(2) INFORMATION FOR SEQ ID NO: 2:

(i) SEQUENCE CHARACTERISTICS:

(A) LENGTH: 23 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 2:

Lys Asn His Lys Asn Leu Leu Lys Ile Ala Gln Tyr Ala Ala Gln Asn
1               5                   10                  15

Arg Arg Gly Leu Asp Leu Leu
            20

(2) INFORMATION FOR SEQ ID NO: 3:

(i) SEQUENCE CHARACTERISTICS:
(A) LENGTH: 24 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 3:

Gly Leu Asp Leu Leu Phe Trp Glu Gln Gly Gly Leu Cys Lys Ala Ile
1               5                   10                  15

Gln Glu Gln Cys Cys Phe Leu Asn
            20

(2) INFORMATION FOR SEQ ID NO: 4:

(i) SEQUENCE CHARACTERISTICS:
(A) LENGTH: 24 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 4:

Leu Asn Thr Glu Pro Ser Gln Leu Pro Pro Thr Ala Pro Pro Leu Leu
1               5                   10                  15

Ser His Ser Asn Leu Asp His Ile
            20

(2) INFORMATION FOR SEQ ID NO: 5:

(i) SEQUENCE CHARACTERISTICS:
(A) LENGTH: 23 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 5:

Leu Leu Ser His Ser Asn Leu Asp His Ile Leu Glu Pro Ser Ile Pro
1               5                   10                  15

Trp Lys Ser Lys Leu Leu Thr
            20

(2) INFORMATION FOR SEQ ID NO: 6:

(i) SEQUENCE CHARACTERISTICS:
(A) LENGTH: 24 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 6:

Gly Leu Asp Leu Leu Phe Trp Glu Gln Gly Gly Leu Cys Lys Ala Leu
1               5                   10                  15

Gln Glu Gln Cys Cys Phe Leu Asn
            20

(2) INFORMATION FOR SEQ ID NO: 7:

    (i) SEQUENCE CHARACTERISTICS:
        (A) LENGTH: 24 amino acids
        (B) TYPE: amino acid
        (C) STRANDEDNESS:
        (D) TOPOLOGY: linear

   (ii) MOLECULE TYPE: peptide

  (iii) HYPOTHETICAL: NO

    (v) FRAGMENT TYPE: internal

   (vi) ORIGINAL SOURCE:
        (A) ORGANISM: Human lymphotropic virus type I

   (xi) SEQUENCE DESCRIPTION: SEQ ID NO: 7:

    Gly Leu Asp Leu Leu Phe Trp Glu Gln Gly Gly Leu Cys Lys Ala Leu
    1               5                   10                  15

    Gln Glu Gln Cys Tyr Phe Leu Asn
                20

(2) INFORMATION FOR SEQ ID NO: 8:

    (i) SEQUENCE CHARACTERISTICS:
        (A) LENGTH: 23 amino acids
        (B) TYPE: amino acid
        (C) STRANDEDNESS:
        (D) TOPOLOGY: linear

   (ii) MOLECULE TYPE: peptide

  (iii) HYPOTHETICAL: NO

    (v) FRAGMENT TYPE: internal

   (vi) ORIGINAL SOURCE:
        (A) ORGANISM: Human lymphotropic virus type I

   (xi) SEQUENCE DESCRIPTION: SEQ ID NO: 8:

    Val Lys Asn His Lys Asn Leu Leu Lys Ile Ala Gln Tyr Ala Ala Gln
    1               5                   10                  15

    Asn Arg Arg Gly Leu Asp Leu
                20

(2) INFORMATION FOR SEQ ID NO: 9:

    (i) SEQUENCE CHARACTERISTICS:
        (A) LENGTH: 23 amino acids
        (B) TYPE: amino acid
        (C) STRANDEDNESS:

(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Human lymphotropic virus type I

(xi) SEQUENCE DESCRIPTION: SEQ ID NO: 9:

Pro Ile Trp Phe Leu Asn Thr Glu Pro Ser Gln Leu Pro Pro Thr Ala
1               5                   10                  15

Pro Pro Leu Leu Pro His Ser
            20